# EUROPEAN PATENT SPECIFICATION

(11) **EP 2 001 308 B1**
(45) Date of publication and mention of the grant of the patent: **15.12.2010**
(21) Application number: 07717721.0
(22) Date of filing: 21.03.2007
(51) Int. Cl.: A23L 1/0522, A23L 1/054, A23L 1/0524, A23L 1/0526, A23L 1/0532, A23L 1/0534, A23L 1/052, A61K 8/73

(54) **PASTEURISATION STABLE STARCH COMPOSITIONS**
PASTEURISIERUNGSSTABILE STÄRKEZUSAMMENSETZUNGEN
COMPOSITIONS D'AMIDON STABLES VIS-À-VIS DE LA PASTEURISATION

(30) Priority: 31.03.2006 EP 06251802
(43) Date of publication of application: 17.12.2008
(73) Proprietor: Cargill Incorporated, Wayzata MN 55391 (US)
(72) Inventor: KETTLITZ, Bernd, Wolfgang, B-2820 Bonheiden (BE); COPPIN, Jozef, Victor, Jean, Marie, B-9470 Denderleeuw (BE); DEBON, Stephane, Jules, Jerome, B-1060 Bruxelles (BE); MEERT, Florent, Francois, Laurent, B-9320 Erembodegem (BE)
(74) Representative: Wilkinson, Stephen John
(86) International application number: PCT/EP2007/052684
(87) International publication number: WO 2007/113111

(56) References cited:
- EP-A- 0 342 738
- WO-A-88/06847
- FR-A- 2 847 770
- GB-A- 2 249 467
- US-A- 3 598 614
- US-A- 4 291 066
- US-A- 4 597 974
- US-A- 5 786 009
- PATENT ABSTRACTS OF JAPAN vol. 2003, no. 12, 5 December 2003 (2003-12-05) & JP 2004 215540 A (Q P CORP), 5 August 2004 (2004-08-05)

## Description

### Technical field

The present invention is in the field of starch-based compositions which exhibit no or low viscosity development during the pasteurisation step.

### Background of the invention

Pasteurisation is an important process in many applications involving starch-based products. The pasteurisation process is commonly carried out by heating. A good heat penetration in aqueous solutions/suspensions during the pasteurisation step is thereby important. The development of viscosity at temperatures between 65°C and 85°C substantially hinders an effective heat penetration and therefore an effective pasteurisation. Starch-based products are commonly employed in the food industry to provide the important viscosity in products, for example in sauces and soups. Conventional starch-based products develop substantial viscosities at pasteurisation temperatures between 65°C and 85°C. Therefore, a reheating of such starch-based products after pasteurisation does not lead to the desired increase in viscosity necessary for the good mouthfeel. Efforts have been undertaken to develop starch-based products that exhibit significant viscosities only above the pasteurisation temperature. Generally, osmotic pressure increasing agents and water-activity depressing agents have been employed to achieve these goals.

EP 0 012 465 reports a way of reducing the rate of swelling of starch in an aqueous starch concentrate during pasteurisation. This is achieved by lowering the osmotic gradient between the interior of the starch granule and the aqueous solution in which the starch is dispersed. The osmotic gradient is lowered by adding suitable compounds such as salts, glycerol, glucose and protein hydrolysates to the concentrate. The addition of hot water to the starch concentrate leads to the decrease in the concentration of these compounds and this again leads to the swelling of the starch.

US 4,291,066 reports a process for producing an aqueous concentrate which can be pasteurised and subsequently reconstituted into a lump-free, thick soup, sauce, gravy or dessert. The process involves producing a concentrate comprising (i) a proportion of starch sufficient to obtain a product of the desired degree of creaminess, (ii) a proportion of an osmotic pressure increasing agent, sufficient to substantially reduce the rate of swelling of starch granules, and (iii) a proportion of a water-activity depressing agent so that the water-activity is not exceeding 0.92.

There is still a need for starch-based compositions which exhibit moderate viscosity at ambient temperature and no or a low viscosity increase at pasteurisation temperatures between 65 and 85°C. The present invention addresses this need by providing such starch-based compositions without having to resort to osmotic pressure increasing and/or water-activity depressing agents.

### Summary of the invention

The present invention relates to a composition that comprises or consist essentially of (a) at least one pregelatinized starch and (b) at least one cook-up starch or flour, wherein the cook-up starch or flour has an amylose content of between 10 and 40% by weight based on the dry substance of the starch or flour. If the cook-up starch or flour is native, then the number average starch granule particle size of the cook-up starch or flour is equal or less than 20 micrometer. In embodiment of the invention, the ratio by weight on dry basis of the two components, (a) and (b), is from 1 : 0.5 to 1 : 3. In another embodiment, the ratio by weight on dry basis of the two components, (a) and (b), more preferably 1 : 1 to 1 : 1.5.

The invention further relates to a liquid composition, a food composition, a soup and sauce formulation comprising the composition according to the invention. Furthermore, the invention also refers to the use of the composition according to the present invention in food, feed, cosmetic, or pharmaceutical applications, in particular where the application involves a pasteurisation step at 65°C to 85°C, preferably 65°C to 75°C.

### Detailed description of the invention

"Pregelatinized starches" - also known as pregelled or instant starches - means starches which yield a viscous paste when dispersed in cold water. Swollen granules in pregelatinised starches may be predominantly intact, e.g. in spray-cooked starches, or partially intact, e.g. in roll-dried starches.

The term "cook-up starch" includes any granular starch that requires water and heat in order to gelatinize and paste. It includes both native and modified starches.

"Modified starch" means any starch which has been treated chemically or physically so as to change the functionality of the starch and to modify one or more of its key physical or chemical properties such as thickening properties, freeze-thaw stability, gel formation, cold water solubility, shear resistant viscosity, and the like. Physically modified starches for example include but are not limited to thermally-inhibited starches.

"Flour" means foodstuffs obtained from the meal of a grain or other starchy food sources. Commonly, flours are powders made from cereal grains or other starchy food sources and which are still containing proteins, such as for example wheat gluten.

The term "native starch" means starch recovered in the original form by extraction from any starch-bearing material.

The number average starch granule particle size is measured by laser diffraction with a Sympatec laser diffraction sensor HELOS/BF.

The amylose content of the starch is determined according to a standard method as described in Morrison, W.R, Laignelet, B., Journal of Cereal Science, 1, 9-20, 1983. The subject matter of which is incorporated by reference.

The water activities were measured on an Aqualab CX-2 from Wilten Instruments at 20° C.

"Cross-bonded starch" within this context means starch which has been treated with a bi- or polyfunctional reagent so that a small number of the starch polymer chains are chemically linked by the cross linking reagent moiety.

"Substituted starch" means starch where substituent groups have been introduced at the hydroxyl groups of the glucose units. Substitution of starches may be via esterification or etherification of the glucose units.

In a first aspect, the present invention relates to a composition which comprises or consists essentially of (a) at least one pregelatinized starch and (b) at least one cook-sup starch or flour, whereby the cook-up starch or flour has an amylose content of between 10 and 40% by weight on the dry substance of the starch or flour, preferably between 10% and 30%, more preferably between 15% and 25%. The cook-up starch of the composition may be modified or native starch. If the cook-up starch or flour is native, then the number average starch granule particle size of said native starch or flour has to be equal or less than 20 micrometer, preferably less than 10 micrometer, more preferably less than 5 micrometer.

This invention is based in part on the surprising findings of the present inventors that when heating aqueous slurries of the composition of the present invention to pasteurisation temperatures between 65°C to 85°C, no or a low viscosity development can be observed. In a particular instance, surprising findings were observed at pasteurisation temperatures of from about 65°C to 75° with respect to low viscosity development. Such a low viscosity development is not expected because in commonly used starch-based compositions the viscosity at any particular temperature is equal to the sum of the viscosities of each starch component when measured separately. Additionally, it has been found that this low viscosity development has no negative effect on the development of the full viscosity at temperatures above 90°C.

The surprising effect of low viscosity development at pasteurisation temperature is illustrated in Example 1 where the viscosity development of a slurry of a starch-based composition containing waxy rice starch is compared with the viscosity development of a slurry of a composition containing rice starch. Both slurries contain the same amount of a cold water soluble spray-cooked starch (2.8% w/w) which gives a Brabender viscosity of about 200 BU at 50°C for both slurries. The concentration of the waxy rice starch and the rice starch was kept the same in both slurries at 3% w/w. Both slurries were heated according to the same temperature profile on a Micro Visco-Amylo Graph® by Brabender®. In order to determine the viscosity development, the Brabender viscosities were measured at 95°C and 70°C during the heating and the ratio of the viscosities (Brabender viscosity at 95°C/ Brabender viscosity at 70°C) calculated. "During the heating" means that the Brabender viscosities are measured first at 70°C and then after further heating at 95°C without any cooling step in between. This ratio gives an indication on one hand of the viscosity development at pasteurisation temperatures between 65°C to 85°C, and on the other hand an indication of the ability to develop full viscosity at higher temperatures. The much higher ratio of the Brabender viscosities at 95°C and 70°C (Brabender viscosity at 95°C/ Brabender viscosity at 70°C) for the slurry containing the rice starch (4.1) than for the slurry containing the waxy rice starch (1.2) indicates that surprisingly only rice starch is pasteurisation stable at temperatures between 65°C to 85°C but not waxy rice starch. Further investigations showed that surprisingly only slurries containing compositions comprising (a) at least one pregelatinized starch and (b) at least one cook-up starch or flour, characterized in that the cook-up starch or flour has an amylose content of between 10% and 40% by weight based on the dry substance of the starch or flour, and wherein, in case that said cook-up starch or flour is native, the number average starch granule particle size of the cook-up starch or flour is equal or less than 20 micrometer, can provide both functions; the functions being, pasteurisation stability between 65°C to 85°C and full viscosity development at higher temperatures. Additionally, a slurry containing a composition according to the present invention has the advantage that the cold water viscosity at 50°C is on one hand big enough to avoid phase separation and precipitation of food ingredients in the slurry, and on the other hand small enough to allow efficient heat transfer within the liquid. Heat transfer is particularly important for the pasteurisation step.

The surprising findings by the present inventors are based in part on the development of a novel method for measuring the viscosity profiles of starches on a Micro Visco-Amylo Graph® by Brabender®. Commonly, the measurements of starch viscosity profiles on a Micro Visco-Amylo Graph® by Brabender® are carried out on a 5.5% w/w starch slurry in water at a pH of 5.5. A problem with this setup is that the gelatinisation temperature of the starch measured does not correspond to the actual gelatinisation temperature and it is therefore not possible to select pasteurisation stable starches from such measurements. Through research in how to improve the measurement methods, it was found that the gelatinisation temperature of starches is more accurately determined by measuring the Brabender viscosity temperature profile of a starch in an aqueous solution of pH 4 to 8 or alternatively, pH 5 to 7, and having a Brabender viscosity at 50°C in the range between 100.and 500 BU, preferably in the range between 100 and 400 BU, more preferably in the range between 100 and 300 BU and most preferably in the range between 150 and 200 BU. These findings were confirmed by differential scanning calorimetry and microscopy. It was found that regular maize starch starts to swell in an aqueous solution at 65°C, whereas no such swelling was found up to 70°C when standard methods were used. "Standard methods" in this context mean any method for measuring the Brabender viscosity / temperature dependence in an aqueous solution having no significant viscosity at 50°C. This new method therefore provides crucial information for selecting starches according to their granule swelling characteristics.

In one embodiment of the present invention, the ratio by weight on dry basis of components (a) and (b) of the composition of the present invention is from 1 : 0.5 to z : 3, preferably 1 : 1 to 1 : 1.5. Furthermore, the composition preferably comprises 33 to 75% of cook-up starch or flour (component (b)) by weight based on the dry substance of the composition.

The first component of the composition according to the present invention is a pregelatinized starch (component (a)). The pregelatinized starch may be both a modified or an unmodified starch, or a mixture of both. Preferred modified pregelatinized starches are n-octenyl succinate starch, cross-bonded - hydroxypropylated starch, cross-bonded - acetylated starch, and mixtures thereof. Preferred unmodified pregelatinized starches are waxy maize starch, regular maize starch, potato starch, tapioca starch, and mixtures thereof. In a particular aspect of the present invention, the pregelatinized starches are made by spray-cooking.

Component (b) of the composition according to the present invention may be a cook-up starch or a flour. The cook-up starch in the composition may be native or modified. Preferred native starches as cook-up starches in the present invention are rice starch, wheat B starch, wheat starch, quinoa starch, sorghum starch, regular maize starch, saponaria vaccaria starch, and mixtures thereof. In a particular embodiment of the invention, compontent (b) is rice starch, wheat B starch, or quinoa starch. Preferred modified starches as cook-up starches in the present invention are cross-bonded, cross-bonded substituted starch, and mixtures thereof. In a particular embodiment of the invention, modified starches are acetylated di-starch adipates and hydroxypropylated di-starch phosphates made from starches selected from the group consisting of regular maize starch, tapioca starch, wheat starch, and mixtures thereof.

The preferred flour to use as component (b) in the composition of the present invention is rice flour.

The addition of emulsifiers may be capable of increasing the ratio of the Brabender viscosities of the compositions in aqueous solution measured at 95°C and 70°C during the heating process. Preferred emulsifiers are anionic such as diacetyl tartaric acid ester of mono-diglycerides (DATEM) and sodium stearoyl lactylate (SSL).

The compositions according to the present invention may be in the form of a dry mix, for example as a powder, or in any other suitable form. This invention relates to a dry mix comprising the composition according to the current invention. The present invention also relates to a liquid composition comprising the composition of the present invention and an edible liquid. Preferred liquid compositions comprise 2% to 7% w/w of the composition of the present invention, preferably from 3% to 5%.

The compositions of the present invention are particularly of interest in food, feed, cosmetic, and pharmaceutical applications, especially whenever there is a requirement for a pasteurisation step and the development of significantly higher viscosity at a further heating step. The invention therefore further relates to a food composition comprising the composition of the present invention and a further food ingredient. Food ingredients include but are not limited to vegetable pieces, fruits, raisins, meat, pasta pieces, and the like. The food composition may be employed in powder form, as a liquid or slurry or in any other suitable form.

The compositions of the present invention are particularly advantageous in the preparation of soups and sauces. Food ingredients present in soups and sauces, for example vegetable or pasta pieces, do not precipitate and agglomerate together at cold temperatures due to the presence of a cold water viscosity. Preferred soup or sauce formulations comprise 2% to 7% w/w the composition of the present invention, preferably from 3% to 5%.

No or low viscosity development of starches can generally be achieved by adding osmotic pressure increasing agents and/or water-activity depressing agents to starch slurries. It has to be understood that it is the object of this invention to provide pasteurisation stable starch-based compositions that do not require such additions. The slurries and other applications comprising the compositions according to the present invention can therefore be characterized that they have a water-activity of more than 0.95, preferably more than 0.97.

The current invention is illustrated in the following examples.

### Example 1:

The Brabender viscosity measurements were carried out on a Micro Visco-Amylo-Graph® by Brabender®. Slurries of (A) 3% rice starch (Remy FG obtained from Remy Industries, Wijgmaal, Belgium) and (B) 3% waxy rice starch (Remy FG obtained from Remy Industries, Wijgmaal, Belgium) by weight on dry basis and 2.8% spray-cooked stabilised n-octenyl succinate waxy maize starch (C*Em Tex 06328 obtained by Cargill, prepared as described in WO 2005/100407 A1) weight on dry basis were prepared with water. The total weight of the slurry was 105 g. The pH was adjusted to 5.5 with 0.1 N NaOH or 0.1 N HCl.

The starch-water slurry was then stirred at 250 rpm and heated according to the following temperature profile:
- starting at 50°C
- rising to 68°C at a rate of 3°C/min
- remaining at 68°C for 105 s
- rising to 95°C at a rate of 3°C/min
- cooling to 50°C at a rate of 3°C/min.

**Table 1: Brabender viscosities at 50°C and ratios of the Brabender viscosities at 95°C and 70°C of compositions (A) and (B) (Brabender viscosity at 95°C/ Brabender viscosity at 70°C) measured according to the above method during the heating process.**

| Cook-up starch/ flour | Pregelatinized starch | Brabender viscosity at 50°C | Brabender viscosity at 95°C/ Brabender viscosity at 70°C | Water activity (a_{w}) |
|---|---|---|---|---|
| Rice starch | C*Em Tex 06328 | 200 | 4.1 | 0.99 |
| Waxy rice starch | C*Em Tex 06328 | 200 | 1.2 | 0.99 |

### Example 2:

A slurry of 3% wheat B starch (obtained from Cargill Wheat Plant at Barby, Germany) by weight on dry basis and 2.8% spray-cooked stabilised n-octenyl succinate waxy maize starch (C*Em Tex 06328 obtained by Cargill, prepared as described in WO 2005/100407 A1) weight on dry basis was prepared with water. The total weight of the slurry was 105 g. The pH was adjusted to 5.5 with 0.1 N NaOH or 0.1 N HCl.

**Table 2 Brabender viscosity at 50°C and ratio of the Brabender viscosities at 95°C and 70°C (Brabender viscosity at 95°C/ Brabender viscosity at 70°C) measured according to the method in Example 1.**

| Cook-up starch/ flour | Pregelatinized starch | Brabender viscosity at 50°C | Brabender viscosity at 95°C/ Brabender viscosity at 70°C | Water activity (a_{w}) |
|---|---|---|---|---|
| Wheat B | C*Em Tex 06328 | 200 | 3.5 | 0.99 |

### Example 3:

A slurry of 3% rice flour (obtained from Cargill Wheat Plant at Barby, Germany) by weight on dry basis and 2.8% spray-cooked stabilised n-octenyl succinate waxy maize starch (C*Em Tex 06328 obtained by Cargill, prepared as described in WO 2005/100407 A1) weight on dry basis was prepared with water. The total weight of the slurry was 105 g. The pH was adjusted to 5.5 with 0.1 N NaOH or 0.1 N HCl.

**Table 3 Brabender viscosity at 50°C and ratio of the Brabender viscosities at 95°C and 70°C (Brabender viscosity at 95°C/ Brabender viscosity at 70°C) measured according to the method in Example 1.**

| Cook-up starch/ flour | Pregelatinized starch | Brabender viscosity at 50°C | Brabender viscosity at 95°C/ Brabender viscosity at 70°C | Water activity (a_{w}) |
|---|---|---|---|---|
| Rice flour | C*Em Tex 06328 | 200 | 4.9 | 0.99 |

### Example 4:

A slurry of 3% rice flour (obtained from Oriental Food Co. Ltd., Bangkok, Thailand, Art. no. 13261) by weight on dry basis, 2.8% spray-cooked stabilised n-octenyl succinate waxy maize starch (C*Em Tex 06328 obtained by Cargill, prepared as described in WO 2005/100407 A1) weight on dry basis and 0.075% Panodan M2010 DATEM emulsifier (obtained from Danisco) by weight on dry basis was prepared with water. The total weight of the slurry was 105 g. The pH was adjusted to 5.5 with 0.1 N NaOH or 0.1 N HCl.

**Table 4 Brabender viscosity at 50°C and ratio of the Brabender viscosities at 95°C and 70°C (Brabender viscosity at 95°C/ Brabender viscosity at 70°C) measured according to the method in Example 1.**

| Cook-up starch/ flour | Pregelatinized starch | Emulsifier | Brabender viscosity at 50°C | Brabender viscosity at 95°C/ Brabender viscosity at 70°C | Water activity (a_{w}) |
|---|---|---|---|---|---|
| Rice flour | C*Em Tex 06328 | Panodan M2010 DATEM (diacetyl tartaric acid ester of mono-diglycerides) | 200 | 6.7 | 0.99 |

### Example 5:

A slurry of 3% rice flour (obtained from Oriental Food Co. Ltd., Bangkok, Thailand, Art. no. 13261) by weight on dry basis, 2.8% spray-cooked stabilised n-octenyl succinate waxy maize starch (C*Em Tex 06328 obtained by Cargill, prepared as described in WO 2005/100407 A1) weight on dry basis and 0.075% Grindsted SSL (sodium stearoyl lactylate) P 55 VEG (obtained from Danisco) by weight on dry basis was prepared with water. The total weight of the slurry was 105 g. The pH was adjusted to 5.5 with 0.1 N NaOH or 0.1 N HCl.

**Table 5 Ratio of the Brabender viscosities of the above composition at 95°C and 70°C (Brabender viscosity at 95°C/ Brabender viscosity at 70°C) measured according to the method in Example 1.**

| Cook-up starch/ flour | Pregelatinized starch | Emulsifier | Brabender viscosity at 50°C | Brabender viscosity at 95°C/ Brabender viscosity at 70°C | Water activity (a_{w}) |
|---|---|---|---|---|---|
| Rice flour | C*Em Tex 06328 | Grindsted SSL (sodium stearoyl lactylate) P 55 VEG | 200 | 5.5 | 0.99 |

### Example 6:

A slurry of 3 % high cross bonded regular maize adipate by weight on dry basis, 2.8% spray cooked stabilised n-octenyl succinate waxy maize starch (C*Em Tex 06328 obtained by Cargill, prepared as described in WO 2005/1004 007 A1) weight on dry basis and 0.075% Panodan M2010 DATEM emulsifier (obtained from Danisco) by weight on dry basis was prepared with water. The total weight of the slurry was 105 g. The pH was adjusted to 5.5 with 0.1 N NaOH or 0.1 N HCl 0.1N.

**Table 6 Ratio of the Brabender viscosities of the above composition at 95°C and 70°C (Brabender viscosity at 95°C/ Brabender viscosity at 70°C) measured according to the method in Example 1.**

| Cook-up starch/ flour | Pregelatinized starch | Emulsifier | Brabender viscosity at 50°C | Brabender viscosity at 95°C/ Brabender viscosity at 70°C | Water activity (a_{w}) |
|---|---|---|---|---|---|
| high cross bonded regular maize adipate | C*Em Tex 06328 | Panodan M2010 DATEM (diacetyl tartaric acid ester of mono-diglycerides) | 200 | 6.4 | 0.99 |

### Example 7:

A slurry of 3 % high cross bonded regular maize adipate by weight on dry basis, 2.8% spray cooked stabilised n-octenyl succinate waxy maize starch (C*Em Tex 06328 obtained by Cargill, prepared as described in WO 2005/1004 007 A1) weight on dry basis and 0.075% Grindsted SSL (sodium stearoyl lactylate) P 55 VEG (obtained from Danisco) by weight on dry basis was prepared with water. The total weight of the slurry was 105 g. The pH was adjusted to 5.5 with 0.1 N NaOH or 0.1 N HCl 0.1N.

**Table 7 Ratio of the Brabender viscosities of the above composition at 95°C and 70°C (Brabender viscosity at 95°C/ Brabender viscosity at 70°C) measured according to the method in Example 1.**

| Cook-up starch/ flour | Pregelatinized starch | Emulsifier | Brabender viscosity at 50°C | Brabender viscosity at 95°C/ Brabender viscosity at 70°C | Water activity (a_{w}) |
|---|---|---|---|---|---|
| high cross bonded regular maize adipate | C*Em Tex 06328 | Grindsted SSL (sodium stearoyl lactylate) P 55 VEG | 200 | 6.0 | 0.99 |

### Example 8:

The following soup formulation was prepared:
A slurry of 1.5 % high cross bonded regular maize adipate by weight on dry basis, 2.8 % spray-cooked stabilised n-octenyl succinate waxy maize starch (C*Em Tex 06328, prepared as described in WO 2005/100407 A1) by weight on dry basis, 11.5 % tomato puree (32 % dry substance) by weight, 1.2 % skimmed milk powder by weight, 0.5 % table salt by weight and 1 % sucrose by weight was prepared with water. The total weight of the slurry was 105 g. The ratio of the Brabender viscosities of the soup at 95°C and 70°C (Brabender viscosity at 95°C/ Brabender viscosity at 70°C) measured during the heating process is shown in Table 8.

**Table 8 Ratio of the Brabender viscosities of the above soup formulation at 95°C and 70°C (Brabender viscosity at 95°C/ Brabender viscosity at 70°C) measured according to the method in Example 1.**

| Cook-up starch/flour | Pregelatinized starch | Brabender viscosity at 50°C | Brabender viscosity at 95°C/ Brabender viscosity at 70°C | Water activity (a_{w}) |
|---|---|---|---|---|
| high cross bonded regular maize adipate | C*Em Tex 06328 | 200 | 2.8 | 0.99 |

## Claims

1. A composition comprising (a) at least one pregelatinized starch and (b) at least one cook-up starch or flour, **characterized in that** the cook-up starch or flour has an amylose content of between 10 and 40% by weight based on the dry substance of the starch or flour, and wherein, in case that said cook-up starch or flour is native, the number average starch granule particle size of the cook-up starch or flour is equal or less than 20 micrometer.

2. The composition according to claim 1 wherein the ratio by weight on dry basis of component (a) to component (b) is from 1 : 0.5 to 1 : 3.

3. The composition according to any one of claims 1 to 2 wherein the composition comprises 33% to 75% of cook-up starch or flour by weight based on the dry substance of the composition.

4. The composition according to any one of claims 1 to 3 wherein the cook-up starch is a native starch selected from the group consisting of rice starch, wheat B starch, wheat starch, quinoa starch, sorghum starch, regular maize starch, saponaria vaccaria starch, and mixtures thereof, preferably rice starch, wheat B starch, quinoa starch.

5. The composition according to any one of claims 1 to 4 wherein the cook-up starch is a modified starch and is selected from the group of cross-bonded, cross-bonded substituted starch, and mixtures thereof.

6. The composition according to claim 5 wherein the modified starch is an acetylated di-starch adipate starch or a hydroxypropylated di-starch phosphate starch selected from the group consisting of regular maize starch, tapioca starch, wheat starch, and mixtures thereof.

7. The composition according to any one of claims 1 to 4 wherein the flour is rice flour.

8. The composition according to any one of claims 1 to 7 wherein the pregelatinized starch is selected from the group consisting of modified pregelatinized starch, unmodified pregelatinized starch, and mixtures thereof.

9. The composition according to claim 8 wherein the pregelatinized starch is a modified starch and selected from the group consisting of n-octenyl succinate starch, cross-bonded - hydroxypropylated starch, cross-bonded - acetylated starch, and mixtures thereof.

10. The composition according to claim 8 wherein the pregelatinized starch is an unmodified pregelatinized starch and selected from the group consisting of waxy maize starch; regular maize starch, potato starch, tapioca starch, and mixtures thereof.

11. The composition according to any one of claims 8 to 10 wherein the pregelatinized starch is spray-cooked starch.

12. Dry mixes comprising the composition according to any preceding claim.

13. A liquid composition comprising the composition according to any preceding claim and an edible liquid.

14. The liquid composition according to claim 13 wherein the composition according to any one of claims 1 to 12 is present from 2% to 7% by weight of the liquid composition.

15. The liquid composition according to claim 13 or 14 wherein the water activity is more than 0.95.

16. A food composition comprising the composition according to any preceding claim and a further food ingredient.

17. A soup or sauce formulation comprising the composition according to any preceding claim.

18. The soup or sauce formulation according to claim 17 wherein the composition according to any one of claims 1 to 12 is present from 2% to 7% by weight of the soup or sauce formulation.

19. The soup or sauce formulation according to claim 17 wherein the water activity is more than 0.95.

20. Use of a composition or dry mix according to any one of claims 1 to 12 in food, feed, cosmetic, and pharmaceutical applications.

21. Use according to claim 20 wherein said food, feed, cosmetic, and pharmaceutical application is pasteurised at 65 to 85°C, preferably 65 to 75°C.

## Patentansprüche

1. Zusammensetzung, umfassend (a) mindestens eine Quellstärke und (b) mindestens eine Kochstärke oder Mehl, **dadurch gekennzeichnet, dass** die Kochstärke oder das Mehl einen Amylosegehalt zwischen 10 und 40 Gew.-%, basierend auf der Trockensubstanz der Stärke oder des Mehls, hat und wobei in dem Fall, dass die Kochstärke oder das Mehl nativ ist, die zahlenmittlere Teilchengröße der Stärkekörnchen der Kochstärke oder des Mehls gleich oder kleiner als 20 Mikrometer ist.

2. Zusammensetzung nach Anspruch 1, wobei das Gewichtsverhältnis auf Trockengewichtsbasis von Komponente (a) zu Komponente (b) 1 : 0,5 bis 1 : 3 beträgt.

3. Zusammensetzung nach einem der Ansprüche 1 bis 2, wobei die Zusammensetzung 33 bis 75 Gew.-%, basierend auf der Trockensubstanz der Zusammensetzung, Kochstärke oder Mehl umfasst.

4. Zusammensetzung nach einem der Ansprüche 1 bis 3, wobei die Kochstärke eine native Stärke, ausgewählt aus der Gruppe, bestehend aus Reisstärke, Weizen-B-Stärke, Weizenstärke, Quinoastärke, Sorghumstärke, normaler Maisstärke, Saponaria-vaccaria-Stärke und Gemischen davon, vorzugsweise Reisstärke, Weizen-B-Stärke, Quinoastärke, ist.

5. Zusammensetzung nach einem der Ansprüche 1 bis 4, wobei die Kochstärke eine modifizierte Stärke ist und aus der Gruppe von vernetzter, vernetzter substituierter Stärke und Gemischen davon ausgewählt ist.

6. Zusammensetzung nach Anspruch 5, wobei die modifizierte Stärke acetylierte Distärkeadipat-Stärke oder hydroxypropylierte Distärkephosphat-Stärke, ausgewählt aus der Gruppe, bestehend aus normaler Maisstärke, Tapiokastärke, Weizenstärke und Gemischen davon, ist.

7. Zusammensetzung nach einem der Ansprüche 1 bis 4, wobei das Mehl Reismehl ist.

8. Zusammensetzung nach einem der Ansprüche 1 bis 7, wobei die Quellstärke aus der Gruppe ausgewählt ist, bestehend aus modifizierter Quellstärke, nicht modifizierter Quellstärke und Gemischen davon.

9. Zusammensetzung nach Anspruch 8, wobei die Quellstärke eine modifizierte Stärke ist und aus der Gruppe ausgewählt ist, bestehend aus n-Octenylsuccinat-Stärke, vernetzter hydroxypropylierter Stärke, vernetzter acetylierter Stärke und Gemischen davon.

10. Zusammensetzung nach Anspruch 8, wobei die Quellstärke eine nicht modifizierte Quellstärke ist und aus der Gruppe ausgewählt ist, bestehend aus Wachsmaisstärke, normaler Maisstärke, Kartoffelstärke, Tapiokastärke und Gemischen davon.

11. Zusammensetzung nach einem der Ansprüche 8 bis 10, wobei die Quellstärke sprühgekochte Stärke ist.

12. Trockenmischungen, umfassend die Zusammensetzung nach einem der vorstehenden Ansprüche.

13. Flüssige Zusammensetzung, umfassend die Zusammensetzung nach einem der vorstehenden Ansprüche und eine essbare Flüssigkeit.

14. Flüssige Zusammensetzung nach Anspruch 13, wobei die Zusammensetzung nach einem der Ansprüche 1 bis 12 bei 2 Gew.-% bis 7 Gew.-% der flüssigen Zusammensetzung vorliegt.

15. Flüssige Zusammensetzung nach Anspruch 13 oder 14, wobei die Wasseraktivität mehr als 0,95 beträgt.

16. Nahrungsmittelzusammensetzung, umfassend die Zusammensetzung nach einem der vorstehenden Ansprüche und einen weiteren Nahrungsmittelbestandteil.

17. Suppen- oder Saucenformulierung, umfassend die Zusammensetzung nach einem der vorstehenden Ansprüche.

18. Suppen- oder Saucenformulierung nach Anspruch 17, wobei die Zusammensetzung nach einem der Ansprüche 1 bis 12 bei 2 Gew.-% bis 7 Gew.-% der Suppen- oder Saucenformulierung vorliegt.

19. Suppen- oder Saucenformulierung nach Anspruch 17, wobei die Wasseraktivität mehr als 0,95 beträgt.

20. Verwendung einer Zusammensetzung oder Trockenmischung nach einem der Ansprüche 1 bis 12 in Nahrungsmittel-, Futter-, kosmetischen und pharmazeutischen Anwendungen.

21. Verwendung nach Anspruch 20, wobei die Nahrungsmittel-, Futter, kosmetische und pharmazeutische Anwendung bei 65 bis 85 °C, vorzugsweise 65 bis 75 °C, pasteurisiert wird.

## Revendications

1. Composition comprenant (a) au moins un amidon prégélatinisé et (b) au mois un amidon ou une farine à cuire, **caractérisée en ce que** l'amidon ou la farine à cuire a une teneur d'amylose entre 10 et 40 % en poids sur la base de la substance sèche d'amidon ou de farine, et dans laquelle, dans le cas où ledit amidon ou farine à cuire est natif, la taille de particule moyenne en nombre des granules d'amidon de l'amidon ou de la farine à cuire est égale ou inférieure à 20 micromètres.

2. Composition selon la revendication 1, dans laquelle le rapport en poids sur la base sèche du composant (a) au composant (b) est de 1:0,5 à 1:3.

3. Composition selon l'une quelconque des revendications 1 à 2, dans laquelle la composition comprend de 33 à 75 % en poids d'amidon ou de farine à cuire sur la base de la substance sèche de la composition.

4. Composition selon l'une quelconque des revendications 1 à 3, dans laquelle l'amidon à cuire est un amidon natif choisi dans le groupe constitué par l'amidon de riz, l'amidon de blé B, l'amidon de blé, l'amidon de quinoa, l'amidon de sorgho, l'amidon de maïs commun, l'amidon de Saponaria vaccaria, et leurs mélanges, de préférence, l'amidon de riz, l'amidon de blé B, l'amidon de quinoa.

5. Composition selon l'une quelconque des revendications 1 à 4, dans laquelle l'amidon à cuire est un amidon modifié et est choisi dans le groupe de l'amidon réticulé, l'amidon substitué réticulé, et leurs mélanges.

6. Composition selon la revendication 5, dans laquelle l'amidon modifié est un amidon du type diadipate d'amidon acétylé ou un amidon du type diphosphate d'amidon hydroxypropylé choisi dans le groupe de l'amidon de maïs commun, l'amidon de tapioca, l'amidon de blé, et leurs mélanges.

7. Composition selon l'une quelconque des revendications 1 à 4, dans laquelle la farine est la farine de riz.

8. Composition selon l'une quelconque des revendications 1 à 7, dans laquelle l'amidon prégélatinisé est choisi dans le groupe constitué par l'amidon prégélatinisé modifié, l'amidon prégélatinisé non modifié, et leurs mélanges.

9. Composition selon la revendication 8, dans laquelle l'amidon prégélatinisé est un amidon modifié et choisi dans le groupe constitué par l'amidon du type succinate de n-octényle, l'amidon réticulé - hydroxypropylé, l'amidon réticulé - acétylé, et leurs mélanges.

10. Composition selon la revendication 8, dans laquelle l'amidon prégélatinisé est un amidon prégélatinisé non modifié et choisi dans le groupe constitué par l'amidon de maïs cireux, l'amidon de maïs commun, l'amidon de pomme de terre, l'amidon de tapioca et leurs mélanges.

11. Composition selon l'une quelconque des revendications 8 à 10, dans laquelle l'amidon prégélatinisé est un amidon cuit et lyophilisé.

12. Mélanges déshydratés comprenant la composition selon l'une quelconque des revendications précédentes.

13. Composition liquide comprenant la composition selon l'une quelconque des revendications précédentes et un liquide comestible.

14. Composition liquide selon la revendication 13, dans laquelle la composition selon l'une quelconque des revendications 1 à 12 est présente à raison de 2 à 7 % en poids de la composition liquide.

15. Composition liquide selon les revendications 13 ou 14, dans laquelle l'activité de l'eau est supérieure à 0,95.

16. Composition alimentaire comprenant la composition selon l'une quelconque des revendications précédentes et un autre ingrédient alimentaire.

17. Formulation de type soupe ou sauce comprenant la composition selon l'une quelconque des revendications précédentes.

18. Formulation de type soupe ou sauce selon la revendication 17, dans laquelle la composition selon l'une quelconque des revendications 1 à 12 est présente à raison de 2 à 7 % en poids de la formulation de type soupe ou sauce.

19. Formulation de type soupe ou sauce selon la revendication 17, dans laquelle l'activité de l'eau est supérieure à 0,95.

20. Utilisation d'une composition ou d'un mélange déshydraté selon l'une quelconque des revendications 1 à 12 dans des applications alimentaires, fourragères, cosmétiques, et pharmaceutiques.

21. Utilisation selon la revendication 20, dans laquelle ladite application alimentaire, fourragère, cosmétique, et pharmaceutique est pasteurisée à 65-85 °C, de préférence, à 65-75 °C.
